# EUROPEAN PATENT SPECIFICATION

(11) **EP 2 542 175 B1**
(45) Date of publication and mention of the grant of the patent: **18.02.2015**
(21) Application number: 09805878.7
(22) Date of filing: 22.12.2009
(51) Int. Cl.: A61B 19/00, A61G 15/12

(54) **A SUPPORT DEVICE THAT PROVIDES SUPPORTING OF THE ARM OF THE OPERATOR**
STÜTZVORRICHTUNG ZUR STÜTZE DES ARMS EINES BEDIENERS
DISPOSITIF DE SUPPORT DESTINÉ À SUPPORTER LE BRAS DE L'OPÉRATEUR

(43) Date of publication of application: 09.01.2013
(73) Proprietor: CELIK, Tunc, Istanbul 34740 (TR)
(72) Inventor: CELIK, Tunc, Istanbul 34740 (TR)
(74) Representative: Yavuzcan, Alev
(86) International application number: PCT/TR2009/000158
(87) International publication number: WO 2011/078813

(56) References cited:
- WO-A1-94/01050
- WO-A1-2009/129287
- DE-A1-102008 015 040
- JP-A- 2009 291 363

## Description

This invention relates to a support device which provides supporting of the arm of the operator.

Surgeons especially dentists have to work by leaning over the patient, their spines bent forward, for a long period of time during the operation. The body staying in this position without use of any support for prolonged periods causes tiredness during the day and thus loss of performance, moreover causes occupational illnesses both in back and neck areas in time.

The state of the art document international patent application WO9816164 discloses a hand supporting platform used in open heart surgeries that supports the hand of the operator.

The state of the art document European patent application EP1669038 discloses a supporting mechanism, provided to support of the arm of the operator during the operation.

Both documents in the state of the art disclose a supporting device adjusted manually, that provides support of the arm or hand of the operator mechanically. The mechanical, manually adjusted supporting mechanisms, having to be adjusted manually, especially during operations, in cases which use of both hands is required, causes loss of time. Furthermore, manual adjustment process hinders sensitive and quick adjustment and also causes distraction and tires the operator in the cases where frequent adjustment is necessary. Consequently, the manual adjustment of the supporting mechanism keeps the supporting mechanism from being flexible, dynamic and user friendly enough.

The state of the art document European patent application EP1611864 discloses a micro surgical operation device that provides functioning of the operational device in the hand of the operator according to the movement of the hand of the operator during micro surgical operations. This document does not disclose an embodiment regarding the support of the hand. Document WO-A-2009/129287 describes an arm rest and discloses the features defined in the preamble of claim 1.

The object of the present invention is the realization of a support device that provides support to the arm of the operator during the operation.

The support device realized in order to attain the objectives of the present invention, explicated in the first claim and the respective claims thereof, comprises a carrier that follows and supports the arm of the operator during operation by the help of sensors mounted on the arm of the operator and the carrier, and a movement mechanism that provides the carrier to move automatically.

Thus, fast and easy support of the arm of the operator is provided by automatic movement of the carrier to the appropriate position by two sensors, one mounted on the carrier and the other mounted on the arm of the operator. This provides working of the surgeons and dentist without the need to contract their neck, back, shoulder and arm muscles to stabilize themselves and thus enables working faster and with less exhaustion. Also, illnesses caused due to the pressure applied on the spine during working for long hours bent forward, will be prevented. The support device forms a support, in between the operator and the patient, that the operator can use during the operation. Moreover the support device supports the hand, arm or the upper body against the gravitational force.

The carrier is in sheet form and produced from a soft material for example from sponge, straphor or the like. Accordingly better grip and support of the limb supported by the carrier during a long lasting operation, is provided.

The motor and the transfer elements located on the movement mechanism are controlled by the control unit. The motor and the transfer elements are chosen the type which would respond to fast and sensitive movements of the movement mechanism that is triggered according to the position of the arm. In the preferred embodiment of the invention the motor is a servo or step type motor.

In the preferred embodiment of the invention the movement mechanism comprises three movement transmission elements that enable the movement of the carrier in three perpendicular axes. The first movement transmission element moves up and down in the vertical plane, the second movement transmission element moves left and right in the horizontal plane and the third movement transmission element moves forward and backward in the horizontal axis. Since each movement transmission element is controlled independent from each other, it is provided that the carrier reaches the desired coordinates precisely in all three axes and that the arm is supported.

In an embodiment of the invention the sensors are proximity, position or presence sensors. Moreover the support device comprises obstacle sensors that are placed around the carrier and that prevents the disturbance of the movement of the carrier due to bumping into objects around the carrier.

In another embodiment of the invention the support device comprises a control panel, located on the main body, through which all the parameters related to the arm, carrier and the movement mechanism can be monitored and the necessary information can be inputted by the operator.

In another embodiment of the present invention the support device comprises a memory located on the control unit, in which parameters regarding some special positions are stored by the operator and that provides control of the movement mechanism and carrier in accordance with these values when required.

The invention provides the automatic movement of the carrier to the appropriate position by the help of sensors attached to the arm and the carrier. Thus, the arm of the operator is supported by the support device in a fast and practical manner. Moreover the experts, like surgeons and dentists who become masters at middle age, are provided to work faster, with less effort and thus avoiding decrease in their performance. Meanwhile they are protected from illnesses caused by working leaning forwards for long hours.

The support device realized in order to attain the aim of the present invention is illustrated in the attached figures, where:
Figure 1 - is the schematic view of a support device.
Figure 2 - is the schematic view of the support device and operation seat.

The elements shown in the figures are numbered as follows:
1. Support device
2. Main body
3. Carrier
4. Movement mechanism
5. First movement transmission element
6. Second movement transmission element
7. Third movement transmission element
8. 80. Sensor
9. Control unit
10. Control panel
11. On-Off trigger
12. Intermediate position trigger

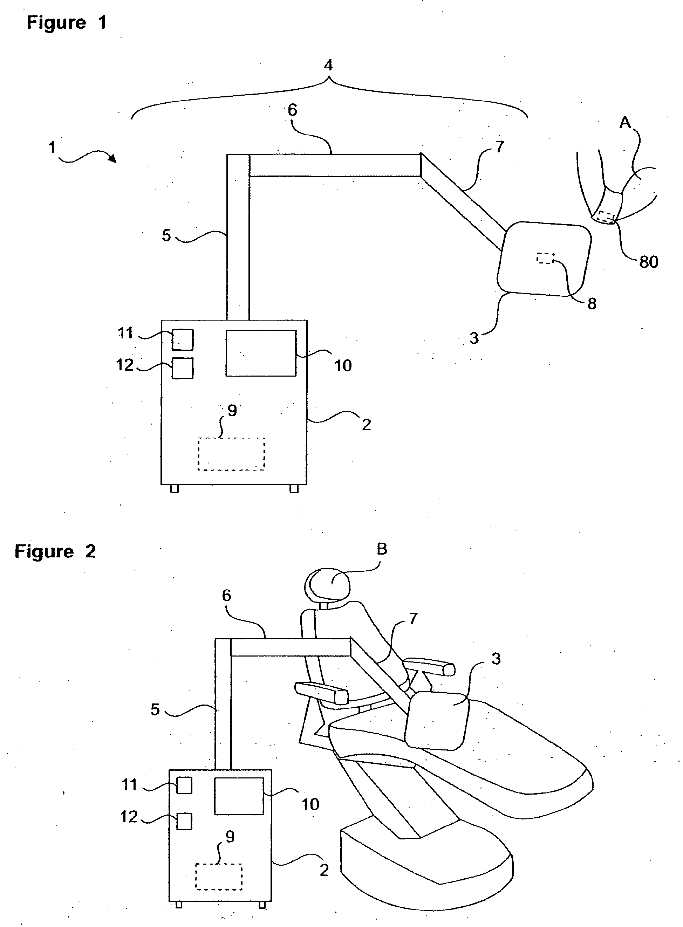

The support device (1) of the present invention comprises, a carrier (3) onto which the arm (A) of the operator is seated, a movement mechanism (4) that provides the movement of the carrier (3), a main body (2) on which the movement mechanism (4) is mounted, at least two sensors (8, 80) one of which is fixed on the arm (A) of the operator and the other is fixed on the carrier (3) and a control unit (9) which provides the carrier (3) to be brought under the arm (A) of the operator (Figure 1).

The support device (1), provides the support of the arm (A) from below by the carrier (3), automatically, without the need for manual adjustment in any way. Hence, surgeons and especially dentists, can perform the operation successfully by leaning over the patient for a long period of time, without getting tired or distracted.

The support device (1) comprises at least one on-off trigger (11) that enables the operator to turn the device on and off which is controlled with hand or feet and at least one intermediate position trigger (12) again controlled by hand or feet.

The support device (1), when turned off has a starting position in which the movement mechanism (4) accumulates minimum space. The support device (1) switches to a working position when it is turned on by the operator triggering the on-off trigger (11). In this position the movement mechanism (4) is activated in accordance with the positions of the sensors (8, 80) and the carrier (3) moves to right below the arm (A) of the operator and stopped by the control unit (9). Unless the operator triggers the on-off or the intermediate position trigger (11 or 12) the support device (1) maintains its position. Further, the support device (1) has an intermediate position. In this intermediate position, with the triggering of the intermediate position trigger (12) by the operator, the movement mechanism (4) moves, for a distance previously determined by the manufacturer or the operator, from the position it was left at its last working position and then stops. Thus, it is provided that the operator switches from one working position to another working position faster and more easily. The support device (1) moves back to the starting position when it is turned off.

The carrier (3) is in sheet form and is mounted on the movement mechanism (4) so that it provides the elbow, the upper arm, the lower arm, the wrist and the hand of the operator to be supported from below. The carrier (3) is pivotally mounted on the movement mechanism (4). Thus, it is provided that the carrier (3) lays in a certain inclination in accordance with the form and the instant position of the limb to be supported. In the preferred embodiment of the application the carrier (3) is manufactured from or covered by a soft material.

The movement mechanism (4) is a robotic arm onto which the carrier (3) is mounted, that provides the up-down and/or left-right and/or forward-backward movement of the carrier (3). The movement mechanism (4) comprises at least one motor controlled by the control unit (9) and transfer elements that provides the transmission of the movement of the motor to the carrier (3). In the preferred embodiment of the invention the movement mechanism (4) that enables the carrier (3) to move on three perpendicular axes, comprises one first movement transmission element (5) that is attached to the main body (2) from one end and that moves on the vertical axes, a second movement transmission element (6) that is perpendicularly pivotally mounted on the other end of the first movement transmission element (5) and which moves to left or right, with reference to the patient seated or lied on the table, in the vertical plane and a third movement transmission element (7) on which to one end the carrier (3) and to the other end the second movement transmission element (6) is pivotally mounted and which moves backward and forward in the horizontal axis. The transfer elements can be gear, gear band or belt pulley. Since the first movement transmission element (5) also carries the other transmission elements, the motor that provides the movement of the first movement transmission element (5) is more powerful than the other motors. The movement transmission elements are preferably telescopic.

The support device (1) comprises a main body (2) which has wheels on its base and can move independently from the operation seat or table (B) (Figure 2). Thus, the support device (1) can be brought to the desired position by the main body (2) being moved by the operator and can be fixed at the desired position. In the preferred embodiment of the invention the triggers (11, 12) are located on the main body (2). In another embodiment of the present invention main body (2) is fixed to the operation seat or table (B). In this embodiment of the present invention the movement mechanism (4) is mounted on the main body (2) which is in the form of an arm (A).

One of the sensors (8, 80) is mounted on the portion of the arm (A) of the operator that is to be supported and the other on the back of the surface of the carrier (3) on which the arm (A) is to be seated. One of these sensor (8, 80) acts as a transmitter and the other acts as a receiver and the sensors sense each other when a previously defined distance in between is reached. The sensor (80) mounted on the arm (A) of the operator is either stick on the arm (A) of the operator or via a wrist band or an armrest. In the preferred embodiment of the invention sensors (8, 80) as placed so as to face each other.

When the support device (1) is activated, the control unit (9) determines the position of the sensors (8, 80) and provides the carrier (3) to be moved right under the arm (A) of the operator such that the sensors (8, 80) will be in the same vertical level and the arm (A) of the operator to be supported when it is seated on the carrier (3). Thus the movement of the carrier (3) under the portion of the arm (A) on which the sensor (8) is mounted and balanced support from below is provided, without being connected to the arm (A) of the operator mechanically.

In the preferred embodiment of the invention one of the sensors (8, 80) is mounted on the arm (A) of the operator and the other is mounted on the carrier (3), preferably in the middle section. When the support device (1) in the starting position is activated by the triggering of the on-off trigger (11) it switches to the working position and the control unit (9) determines the position information from both sensors (8, 80) fixed to the operator's arm (A) and the carrier (3). The control unit (9) controls the movement mechanism (4) according to this position information, so that the carrier (3) moves below the arm (A) to perform the carrying function. The control unit (9) primarily moves the movement mechanism (4) such that both sensors (8, 80) are in the same vertical level and hence provides the carrier (3) to be moved right under the arm (A) of the operator and stops at that position. In an embodiment of the present invention the movement mechanism (4) works in the following manner: Motors, to which each movement transmission element is connected to, are activated and movement of the motors are transmitted to each of the movement transmission elements separately by the transfer elements. Thus, the carrier (3) is moved right below the arm (A) according to the position information received from the sensors (8, 80), such that the sensor (8) fixed to the carrier (3) and the sensor (80) fixed to the arm (A) are on the same level. The motor is stopped by the control unit (9) when the carrier (3) reaches right below the arm (A) and the switch of the movement mechanism (4) from this position to another position is prevented unless the triggers (11, 12) are triggered by the operator. Unless the operator triggers one of the triggers (11, 12) the movement mechanism (4) does not move and the carrier (3) maintains its position, remains stable in that position and supports the arm (A).

In another embodiment of the invention the motor is a servo or a step type motor. Hence precise and minor movements of the movement mechanism (4) are enabled.

In another embodiment of the invention the sensor (8, 80) is a proximity, position or presence sensor.

In another embodiment of the present invention the support device (1) comprises one or more then one obstacle sensors that are positioned around the carrier (3) and/or the movement mechanism (4) and that send warning signals to the control unit (9) to avoid hitting or getting close to the articles around the movement mechanism (4). When the obstacles sensors are activated the movement mechanism (4) is immediately stopped by the control unit (9) and it is kept from moving further.

In another embodiment of the present invention one end of the first movement transmission element (5) is attached to the main body (2). The second movement transmission element (6) is pivotally attached to the other end of the first movement transmission element (5). The second movement transmission element (6), moves with the arm (A) of the operator and a carrier (3) is attached to its one end. In this embodiment the motor moves the first movement transmission element (5) forward and backward. The second movement transmission element (6), on the other hand, moves with the guidance of the arm (A) that seats on the carrier (3). Thus, the raising and lowering of the carrier (3) in the vertical direction by the first movement transmission element (5) automatically and at the same time left-right movement of the carrier (3) in the horizontal plane by the second movement transmission element (6) is provided.

In another embodiment of the invention one end of the first movement transmission element (5) is fixed to the main body (2), whereas the second movement transmission element (6) is fixed to the other end of the first transmission element (5) and the carrier (3) is fixed to the end of the second movement transmission element (6). In this embodiment there exists a motor that moves the first movement transmission element (5) forward and backward and a second motor which is located in between the first and the second movement transmission elements (5 and 6) and that moves the second movement transmission element (6). Therefore the lowering and rising of the carrier (3) in the vertical plane by the first movement transmission element (5) is provided as well as the left and right movement of the carrier (3) by the second movement transmission element (6).

In another embodiment of the present invention support device (1) comprises a break mechanism that prevents possible position changes of the movement mechanism (4) when a load is exerted on the carrier (3). The break mechanism, stops all the motors and provides the carrier (3) to stay at its last position, unless an activation signal is given by the operator.

In another embodiment of the invention the support device (1) comprises a control panel (10) located on the main body (2). Via the control panel (10) all the parameters related to the arm (A), carrier (3) and the movement mechanism (4) can be monitored and the necessary information can be inputted by the operator. In the preferred embodiment of the invention the control panel (10) has a touch screen display.

In an embodiment of the invention the triggers (11, 12) are in the form of manually controlled buttons.

In another embodiment of the invention the triggers (11, 12) are a pedal controlled by feet.

In another embodiment of the invention the triggers (11, 12) are controlled by voice.

In another embodiment of the invention the support device (1) comprises a memory located on the control unit (9). The operator stores in this memory parameters regarding some special positions and it is provided that the carrier (3) and the movement mechanism (4) are controlled in accordance with these parameters when necessary.

In another embodiment of the present invention the support device (1) comprises two or more sensors (80) attached to the portions of the arm (A) that are desired to be supported. Operator introduces every sensor attached to his arm (A) to the control unit (9) and thus movement of the carrier (3) below the portion of the arm (A) where the selected sensor is attached, in accordance with the sensor (80) selected by the operator and support of the said portion is provided.

In another embodiment of the invention the support device (1) comprises a calibration sensor placed on the main body (2). The calibration sensor provides the calibration of the deviations that might occur due to mechanical wear out and collisions during the activity of the support device (1) and the calibration of the distance between the two sensors (8, 80) set by the manufacturer and the operator. Hence, the correctness of the distance between the sensor (8) mounted on the carrier (3) and the sensor (80) attached to the arm (A) of the operator is sensed and it is guaranteed that the support device (1) provides service at the best position.

In another embodiment of the invention the support device (1) comprises a guide that extends towards the top of the operation table or seat (B). The guide, acts as the first movement transmission element (5) and the second movement transmission element (6) is attached to this guide and it hangs down towards the operation table or seat (B). The carrier (3) is mounted on to the second or the third movement transmission element (6 or 7) that hangs down. Hence ergonomic use of the support device (1) is provided.

By this invention the back and neck illnesses, that might occur due to operating by leaning over the patient, their spines bent forward, for a long period of time, are avoided and longer hours of working leaning towards the patient, and completing the operation without getting tired and distracted becomes possible. As the support device (1) work automatically, independent of the operator, just according to the movement of the arm (A), the operator's arm (A) can be supported while he is focused on the patient, without being distracted.

## Claims

1. A support device (1) that comprises a carrier (3) onto which the arm (A) of the operator is seated, a movement mechanism (4) that provides the movement of the carrier (3), a main body (2) on which the movement mechanism (4) is mounted and **characterized by**
a. at least two sensors (8, 80) one of which is fixed on the arm (A) of the operator and the other is fixed on the carrier (3) and
b. a control unit (9) which provides the carrier (3) to be brought under the arm (A) of the operator.

2. A support device (1) in accordance with Claim 1, **characterized by** at least one on-off trigger (11) that enables the operator to turn the device on and off which is controlled with hand or feet and at least one intermediate position trigger (12) again controlled by hand or feet.

3. A support device (1) in accordance with Claim 1 or 2, **characterized by** a control unit (9) that provides the support device (1) to be switched to
a. a starting position in which it accumulates minimum space, when the support device (1) is off,
b. a working position in which the movement mechanism (4) is activated in accordance with the positions of the sensors (8, 80) and the carrier (3) moves to right below the arm (A) of the operator and stops, when the support device (1) is turned on by the operator,
c. an intermediate position in which, with the trigger by the operator, the movement mechanism (4) moves, for a distance previously determined by the manufacturer or the operator, from the position it was left at its last working position and then stops.

4. A support device (1) in accordance with Claim 1, **characterized in that** the carrier (3) is in sheet form and is mounted on the movement mechanism (4) so that it provides the elbow, the upper arm, the lower arm, the wrist and the hand of the operator to be supported from below.

5. A support device (1) in accordance with Claim 1, **characterized in that** the movement mechanism (4) comprises at least one motor controlled by the control unit (9) and transfer elements that provides the transmission of the movement of the motor to the carrier (3).

6. A support device (1) in accordance with Claim 1, **characterized in that** the main body (2) has wheels on its base and can move independently from the operation seat or table (B).

7. A support device (1) in accordance with Claim 1, **characterized in that** the main body (2) is fixed to the operation seat or table (B) and is in the form of an arm (A), on to which the movement mechanism (4) is mounted.

8. A support device (1) in accordance with any one of the claims above, **characterized in that** the movement mechanism (4) that enables the carrier (3) to move on three perpendicular axes, comprises one first movement transmission element (5) that is attached to the main body (2) from one end and that moves on the vertical axes, a second movement transmission element (6) that is perpendicularly pivotally mounted on the other end of the first movement transmission element (5) and which moves to left or right in the vertical plane and a third movement transmission element (7) on which to one end the carrier (3) and to the other end the second movement transmission element (6) is pivotally mounted and which moves backward and forward in the horizontal axis.

9. A support device (1) in accordance with any one of the claims above, **characterized in that** one of the sensors (8, 80) is mounted on the portion of the arm (A) of the operator that is to be supported and the other on the back of the surface of the carrier (3) on which the arm (A) is to be seated.

10. A support device (1) in accordance with Claim 1 or 9, **characterized in that** the sensor (8, 80) is a proximity, position or presence sensor.

11. A support device (1) in accordance with Claim 1, **characterized by** one or more then one obstacle sensors that are positioned around the carrier (3) and/or the movement mechanism (4) and that send warning signals to the control unit (9) to avoid hitting or getting close to the articles around the movement mechanism (4).

12. A support device (1) in accordance with Claim 8, **characterized in that** the movement mechanism (4) comprises a first movement transmission element (5) one end of which is attached to the main body (2) and a second movement transmission element (6) that is pivotally attached to the other end of the first movement transmission element (5), wherein the said second movement transmission element (6), moves with the arm (A) of the operator and a carrier (3) is attached to its other end.

13. A support device (1) in accordance with Claim 8, **characterized in that** the movement mechanism (4) comprises,
a. a first movement transmission element (5) that is fixed to the main body (2),
b. a second movement transmission element (6) on to one end of which the other end of the first transmission element (5) is fixed and on to the other end of which the carrier (3) is fixed,
c. a motor that moves the first movement transmission element (5) forward and backward and
d. a second motor which is located in between the first and the second movement transmission elements (5 and 6) and that moves the second movement transmission element (6).

14. A support device (1) in accordance with Claim 1, **characterized by** a break mechanism that prevents possible position changes of the movement mechanism (4) when a load is exerted on the carrier (3).

15. A support device (1) in accordance with any one of the claims above, **characterized by** two or more sensors (80) attached to the portions of the arm (A) that are desired to be supported.

## Patentansprüche

1. Stützvorrichtung (1), umfassend einen Träger (3), auf dem der Arm (A) des Bedieners ruht, einen Bewegungsmechanismus (4), der die Bewegung des Trägers (3) ermöglicht, einen Hauptkörper (2), an dem der Bewegungsmechanismus (4) angebracht ist, und **gekennzeichnet durch**
a. wenigstens zwei Sensoren (8, 80), von denen einer am Arm (A) des Bedieners und der andere am Träger (3) befestigt ist, und
b. eine Steuereinheit (9), die es ermöglicht, den Träger (3) unter den Arm (A) des Bedieners zu bringen.

2. Stützvorrichtung (1) nach Anspruch 1, **gekennzeichnet durch** wenigstens einen Ein-Aus-Betätiger (11), der es dem Bediener ermöglicht, die Vorrichtung ein- und auszuschalten, und der mit Hand oder Fuß gesteuert wird, und wenigstens einen Zwischenpositionsbetätiger (12), der wiederum mit Hand oder Fuß gesteuert wird.

3. Stützvorrichtung (1) nach Anspruch 1 oder 2, **gekennzeichnet durch** eine Steuereinheit (9), die es ermöglicht, dass die Stützvorrichtung (1) umgeschaltet wird in
a. eine Ausgangsposition, in der sie minimalen Raum akkumuliert, wenn die Stützvorrichtung (1) ausgeschaltet ist,
b. eine Arbeitsposition, in der der Bewegungsmechanismus (4) gemäß den Positionen der Sensoren (8, 80) aktiviert wird und der Träger (3) sich direkt unter den Arm (A) des Bedieners bewegt und anhält, wenn die Stützvorrichtung (1) vom Bediener eingeschaltet wird,
c. eine Zwischenposition, in die sich der Bewegungsmechanismus (4) mit der Betätigung durch den Bediener für eine im Voraus vom Hersteller oder dem Bediener festgelegte Strecke aus der Position bewegt, in der er an seiner letzten Arbeitsposition zurückgelassen wurde, und dann anhält.

4. Stützvorrichtung (1) nach Anspruch 1, **dadurch gekennzeichnet, dass** der Träger (3) bahnförmig ist und derart an dem Bewegungsmechanismus (4) angebracht ist, dass er dafür sorgt, dass der Ellbogen, der Oberarm, der Unterarm, das Handgelenk und die Hand des Bedieners von unten abgestützt werden.

5. Stützvorrichtung (1) nach Anspruch 1, **dadurch gekennzeichnet, dass** der Bewegungsmechanismus (4) wenigstens einen Motor umfasst, der von der Steuereinheit (9) gesteuert wird, und Übertragungselemente, die die Übertragung der Bewegung des Motors auf den Träger (3) ermöglicht.

6. Stützvorrichtung (1) nach Anspruch 1, **dadurch gekennzeichnet, dass** der Hauptkörper (2) Räder an seiner Basis aufweist und sich unabhängig vom Bedienungssitz oder -tisch (B) bewegen kann.

7. Stützvorrichtung (1) nach Anspruch 1, **dadurch gekennzeichnet, dass** der Hauptkörper (2) am Bedienungssitz oder -tisch (B) befestigt ist und die Form eines Arms (A) aufweist, an dem der Bewegungsmechanismus (4) angebracht ist.

8. Stützvorrichtung (1) nach einem der vorangehenden Ansprüche, **dadurch gekennzeichnet, dass** der Bewegungsmechanismus (4) es dem Träger (3) ermöglicht, sich an drei senkrechten Achsen zu bewegen und ein erstes Bewegungsübertragungselement (5), das von einem Ende am Hauptkörper (2) angebracht ist und sich an den vertikalen Achsen bewegt, ein zweites Bewegungsübertragungselement (6), das senkrecht drehbar an dem anderen Ende des ersten Bewegungsübertragungselements (5) angebracht ist und sich in der vertikalen Ebene nach links oder rechts bewegt, und ein drittes Bewegungsübertragungselement (7) umfasst, an dem das eine Ende des Trägers (3) und das andere Ende des zweiten Bewegungsübertragungselements (6) drehbar angebracht sind und das sich an der horizontalen Achse vor und zurück bewegt.

9. Stützvorrichtung (1) nach einem der vorangehenden Ansprüche, **dadurch gekennzeichnet, dass** einer der Sensoren (8, 80) an dem Abschnitt des Arms (A) des Bedieners angebracht ist, der abgestützt werden soll, und der andere an der Rückseite der Fläche des Trägers (3), auf der der Arm (A) ruhen soll.

10. Stützvorrichtung (1) nach Anspruch 1 oder 9, **dadurch gekennzeichnet, dass** der Sensor (8, 80) ein Näherungs-, Positions- oder Anwesenheitssensor ist.

11. Stützvorrichtung (1) nach Anspruch 1, **gekennzeichnet durch** einen oder mehrere Hindernissensoren, die um den Träger (3) und/oder den Bewegungsmechanismus (4) herum angeordnet sind und Warnsignale an die Steuereinheit (9) senden, um ein Stoßen gegen oder ein Annähern an Gegenstände um den Bewegungsmechanismus (4) herum zu vermeiden.

12. Stützvorrichtung (1) nach Anspruch 8, **dadurch gekennzeichnet, dass** der Bewegungsmechanismus (4) ein erstes Bewegungsübertragungselement (5), dessen eines Ende am Hauptkörper (2) angebracht ist, und ein zweites Bewegungsübertragungselement (6) umfasst, das drehbar am anderen Ende des ersten Bewegungsübertragungselements (5) angebracht ist, wobei das zweite Bewegungsübertragungselement (6) sich mit dem Arm (A) des Bedieners bewegt und ein Träger (3) an seinem anderen Ende angebracht ist.

13. Stützvorrichtung (1) nach Anspruch 8, **dadurch gekennzeichnet, dass** der Bewegungsmechanismus (4) Folgendes umfasst:
a. ein erstes Bewegungsübertragungselement (5), das am Hauptkörper (2) befestigt ist,
b. ein zweites Bewegungsübertragungselement (6), an dessem einem Ende das andere Ende des ersten Bewegungsübertragungselements (5) befestigt ist, und an dessen anderem Ende der Träger (3) befestigt ist,
c. einen Motor, der das erste Bewegungsübertragungselement (5) vor und zurück bewegt, und
d. einen zweiten Motor, der zwischen dem ersten und zweiten Bewegungsübertragungselement (5 und 6) angeordnet ist und das zweite Bewegungsübertragungselement (6) bewegt.

14. Stützvorrichtung (1) nach Anspruch 1, **gekennzeichnet durch** einen Unterbrechungsmechanismus, der mögliche Positionsänderungen des Bewegungsmechanismus (4) verhindert, wenn eine Last auf den Träger (3) ausgeübt wird.

15. Stützvorrichtung (1) nach einem der vorstehenden Ansprüche, **gekennzeichnet durch** zwei oder mehrere Sensoren (80), die an den Abschnitten des Arms (A) angebracht sind, die abgestützt werden sollen.

## Revendications

1. Un appareil de soutien (1) comprenant un porteur (3) sur lequel les armes (A) de l'operateur sont placés, un mechanisme du mouvement (4) fournissant le movement de porteur (3), un corps principal (2) sur lequel le mechanisme du mouvement (4) est monté et characterize par;
a. au moins deux détecteurs (8, 80), qu'un des deux est fixé sur le bras (A) de l'operateur et l'autre est fixé sur le porteur (3) et
b. une unité de contrôle qui fournis le porteur (3) etre apporté sous le bras (A) de l'operateur.

2. Un appareil de soutien (1) en conformité avec la Demande 1, est characterisé par au moins une gachette de marche-arret (11) qui permet l'operateur de mettre l'appareil en marche ou en arret, qui peut etre reglé soi avec la main ou avec le pieds et au moins une gachette (12) de position intermédiaire encore reglé par la main ou le pieds.

3. Un appareil de soutien (1) en conformité avec les Demandes 1 ou 2, est characterisé par une unitée de contrôle (9) qui fournis l'appareil de soutien (1) d'être changé a,
a. Une position de début auquel il s'occupe le minimum espace, quandl'appareil de soutien (1) est fermé,
b. Une position de travail auquel le mechanisme du mouvement (4) est activé en conformité avec les positions des détecteurs (8, 80) et du porteur (3) se déplace precisement juste au-dessous du bras (A) de l'operateur et arrête, quand l'appareil de support (1) est activé par l'operateur.
c. Une position intermédiaire dans laquelle, avec le déclanchement par l'opérateur, le mechanisme du mouvement (4) deplace, pour une distance determinée au paravant par le fabricant ou par l'operateur, de la position qu'il avait êté laissé à sa dernière position de travail et s'arrête.

4. Un appariel de soutien (1) en conformité avec la Demande 1, est characterisé puisque le porteur (3) a une forme de plaque et monté sur le mechanisme du mouvement (4) afin de fournir au coude, l'arrière-bras, l'avant-bras, le poignet et la main de l'opérateur d'être soutenu du-dessous.

5. Un appareil de soutien (1) en conformité avec La Demande 1, est characterisé puisque le mechanisme du mouvement (4) comprends au moins un moteur reglé par l'unitée de côntrole (9) et des éléments de transfert qui fournissent la transmission du movement du moteur au porteur (3).

6. Un appareil de soutien (1) en conformite avec La Demande 1, est characterisé puisque le corps principal (2) a des roues dans sa partie inférieur et peut déplacer indépendamment de la fauteuille ou de la table d'opération (B)

7. Un appareil de soutien (1) en conformité avec La Demande 1, est characterisé puisque le corps principal (2), est fixé a la fauteuille ou a la table d'opération et a une forme d'un bras (A) sur lequel le mechanisme du mouvement (4) est monté.

8. Un appareil de soutien (1) en conformité avec tous les demandes citées au-dessus, est characterisé puisque le mechanisme du mouvement (4) qui permet le porteur (3) de se deplacer sur les trois coordonnés, chacune perpandiculaire a l'autre, comprends le premier élément (5) de transmission du mouvement attaché au corps principal (2) d'un des bouts et celui-ci se deplace sur les coordonnés verticales, un deuxième élément (5) de transmission du mouvement qui est attaché perpandiculairement pivotante a l'autre but du premier élément (5) de transmission du mouvement, et qui se deplace a gauche ou a droit dans le plan vertical et un troisième élément (7) de transmission du movement sur lequel en un des bouts le porteur (3) et en autre bout le deuxième élément (6) de transmission du movement est attaché pivotante et qui se deplace en avant et en arrière dans le plan horizontale.

9. Un appareil de soutien (1) en conformité avec tous les demandes citées au-dessus, est characterisé puisque un des détecteurs (8, 80) est attaché sur la partie du bras (A), de l'operateur qui va être soutenu et l'autre sur l'arrière de la surface du porteur (3) sur lequel le bras (A) va être placé.

10. Un appareil de soutien (1) en conformité avec la Demande 1 ou 9, est characterisé puisque le détecteur (8, 80) est un détecteur de la proximité, position ou presence.

11. Un appareil de soutien (1) en conformité avec la Demande 1, est characterisé par un ou plus d'un détecteur d'obstacle qui sont placés autour du porteur (3) et/ou le mechanisme du mouvement (4) et qui envoie des signals d'alert a l'unité de côntrole (9) pour empecher la collision ou une approache excessive aux objets autour du mechanisme du mouvement (4).

12. Un appareil de soutien (1) en conformité avec la Demande 8, est characterisé puisque le mechanisme du mouvement, (4), comprends un premier élément (5) de transmission du mouvement qu'un de ses bouts est attaché au corps principal (2) et un deuxième élément (5) de transmission du movement, tandis que deuxième élément (6) de transmission du movement cité, se deplace avec le bras (A) de l'operateur et le porteur (3), est attaché a l'autre bout.

13. Un appareil de soutien (1) en conformité avec la Demande 8, est characterisé par un mechanisme (4) du movement, comprends;
a. un premier élément (5) de transmission qui est fixé au corps (2) principale,
b. un deuxième élément (6) de transmission sur l'un de ses bout, est fixé l'autre bout du premier élément (5) de transmission et que sur l'autre bout le porteur (3) est fixé,
c. un moteur qui se deplace le premier élément (6) de transmission en avant ou en arrière et
d. un deuxième moteur qui est placé entre les premier et deuxième éléments (5 et 6) de transmission et qui fait bouger le deuxième élément (6) de transmission.

14. Un appareil de soutien (1) en conformité avec la Demande 1, est characterisé par un mechanisme de freinage qui empêche un changement de position potentiel du mechanism du mouvement, quand un charge est exercé sur le porteur (3).

15. Un appareil de soutien (1) en conformité avec toutes les Demandes citées au-dessus, est characterisé par par deux ou plus de détecteurs (80) attachés aux partis du bras (A) qu'on voudra etre soutenu.
